# EUROPÄISCHE PATENTANMELDUNG

(11) **EP 3 754 008 A1**
(43) Veröffentlichungstag der Anmeldung: **23.12.2020**
(21) Anmeldenummer: 20178451.9
(22) Anmeldetag: 05.06.2020
(51) Int. Cl.: C11D 3/50, A61K 8/49, A61Q 13/00, C11B 9/00, A61L 9/01

(54) **CYCLISCHE HYDRAZIDE ALS DUFTVORLÄUFERVERBINDUNGEN**

(30) Priorität: 21.06.2019 DE 102019116765
(71) Anmelder: Henkel AG & Co. KGaA, 40589 Düsseldorf (DE)
(72) Erfinder: SCHAEFER, Sascha, 40822 Mettmann (DE); LEDERMANN, Nadia, 47053 Duisburg (DE)
(74) Vertreter: Viering, Jentschura & Partner mbB Patent- und Rechtsanwälte

(57) **Zusammenfassung**

Die vorliegende Erfindung betrifft spezielle Duftvorläuferverbindungen der Formel (I), die sich von Hydraziden und Duftstoffaldehyden und -ketonen ableiten sowie ein Verfahren zu deren Herstellung. Ferner betrifft die vorliegende Erfindung Wasch- oder Reinigungsmittel, kosmetische Mittel sowie Luftpflegemittel, welche solche Duftvorläuferverbindungen enthalten. Des Weiteren betrifft die vorliegende Erfindung ein Verfahren zur lang anhaltenden Beduftung von Oberflächen unter Verwendung der erfindungsgemäßen Duftvorläuferverbindungen und Mittel.

## Beschreibung

Die vorliegende Erfindung betrifft spezielle Duftvorläuferverbindungen der Formel (I), die sich von Hydraziden und Duftstoffaldehyden und -ketonen ableiten sowie ein Verfahren zu deren Herstellung. Ferner betrifft die vorliegende Erfindung Wasch- oder Reinigungsmittel, kosmetische Mittel sowie Luftpflegemittel, welche solche Duftvorläuferverbindungen enthalten. Des Weiteren betrifft die vorliegende Erfindung ein Verfahren zur langanhaltenden Beduftung von Oberflächen unter Verwendung der erfindungsgemäßen Duftvorläuferverbindungen und Mittel.

Wasch- oder Reinigungsmittel bzw. kosmetische Mittel enthalten zumeist Duftstoffe, die den Mitteln einen angenehmen Geruch verleihen. Die Duftstoffe maskieren dabei zumeist den Geruch der anderen Inhaltstoffe, so dass beim Verbraucher ein angenehmer Geruchseindruck entsteht.

Insbesondere im Bereich Waschmittel sind Duftstoffe wichtige Bestandteile der Zusammensetzung, da die Wäsche sowohl im feuchten als auch im trockenen Zustand einen angenehmen und möglichst auch frischen Duft aufweisen soll. Die Verwendung von Duftstoffen ist grundsätzlich problematisch, da es sich bei diesen um mehr oder minder leicht flüchtige Verbindungen handelt, aber dennoch ein langanhaltender Dufteffekt angestrebt wird. Insbesondere bei denjenigen Riechstoffen, die die frischen und leichten Noten des Parfüms darstellen und infolge ihres hohen Dampfdrucks besonders flüchtig sind, ist die gewünschte Langlebigkeit des Dufteindrucks kaum erreichbar.

Eine verzögerte Duftfreisetzung kann z.B. durch sogenannte Duftvorläuferverbindungen erfolgen. Diese Duftvorläuferverbindungen basieren auf Duftstoffverbindungen, insbesondere Duftstoffaldehyden oder -ketonen, welche mit weiteren Verbindungen umgesetzt werden, die in der Lage sind die eigentliche Duftstoffverbindung verzögert durch Hydrolyse oder photochemisch wieder freizusetzten. Obwohl sowohl Duftstoffaldehyde und -ketonen als Ausgangsmaterialien für Duftvorläuferverbindungen beschrieben wurden, sind bis dato keine kommerziell geeigneten Duftvorläuferverbindungen mit Ketonen. Die Aufgabe der vorliegenden Erfindung war deshalb die Bereitstellung von Duftvorläuferverbindungen, insbesondere auf Basis von Duftstoffketonen, welche durch eine einfache Synthese zu erhalten sind. Ferner sollen die Duftvorläuferverbindungen bevorzugt für den Einsatz in Konsumgütern, insbesondere Wasch- und Reinigungsmitteln geeignet sein.

Die Erfinder der vorliegenden Erfindung haben gefunden, dass die Aufgabe durch die speziellen Duftvorläuferverbindungen der Formel (I), die sich von Hydraziden und Duftstoffaldehyden und - ketonen ableiten gelöst werden kann. Überraschend wurde gefunden, dass durch den Einsatz eines cyclischen Hydrazids die unerwünschte Abspaltung von Hydrazin vermieden werden kann.

In einem Aspekt betrifft die Erfindung deshalb eine Duftvorläuferverbindung der Formel (I) 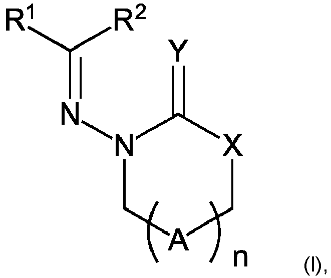 wobei
i) R¹ und R² unabhängig voneinander ausgewählt sind aus H, oder linearen, verzweigten oder cyclischen, gesättigten oder ungesättigten, substituierten oder unsubstituierten Kohlenwasserstoffresten, die bis zu 20 Kohlenstoffatome und gegebenenfalls bis zu 6 Heteroatome enthalten;
   oder
ii) R¹ und R² gemeinsam mit dem Kohlenstoffatom an das sie gebunden sind einen cyclischen, gesättigten oder ungesättigten, Kohlenwasserstoffring mit bis zu 12 Kohlenstoffatomen bilden, der bis zu 6 Heteroatome enthalten kann;
   X und Y unabhängig voneinander ausgewählt sind aus O, S, Se oder NR³, wobei R³ H oder ein linearer oder verzweigter, gesättigter oder ungesättigter, substituierter oder unsubstituierter Kohlenwasserstoffrest mit bis zu 20 Kohlenstoffatomen ist, der gegebenenfalls bis zu 6 Heteroatome enthalten kann;
   A gleich -CH₂-, -CHR⁴-, -CR⁴R⁵- oder ein Heteroatom ist, wobei R⁴ und R⁵ unabhängig voneinander ausgewählt sind aus linearen oder verzweigten, gesättigten oder ungesättigten, substituierten oder unsubstituierten Kohlenwasserstoffresten, die bis zu 20 Kohlenstoffatome und gegebenenfalls bis zu 6 Heteroatome enthalten, wobei falls mehrere Einheiten A vorhanden sind, diese unabhängig voneinander ausgewählt werden,
   n = 0 bis 4 ist,
   mit der Maßgabe, dass sich der Rest R¹-C-R² von einem Duftstoffaldehyd oder einem Duftstoffketon der Formel R¹-C(=O)-R² ableitet, bevorzugt einem Duftstoffketon.

In einem zweiten Aspekt betrifft die vorliegende Erfindung ein Wasch- oder Reinigungsmittel, enthaltend mindestens eine Duftvorläuferverbindung der vorliegenden Erfindung.

In einem dritten Aspekt betrifft die vorliegende Erfindung ein Luftpflegemittel, enthaltend mindestens eine Duftvorläuferverbindung der vorliegenden Erfindung.

In einem vierten Aspekt betrifft die vorliegende Erfindung ein kosmetisches Mittel, enthaltend mindestens eine Duftvorläuferverbindung der vorliegenden Erfindung.

In einem fünften Aspekt betrifft die vorliegende Erfindung ein Verfahren zur Herstellung von Duftvorläuferverbindungen der Formeln (I) gemäß der vorliegenden Erfindung, umfassend das Umsetzen einer Verbindung nach der allgemeinen Formel (IV) 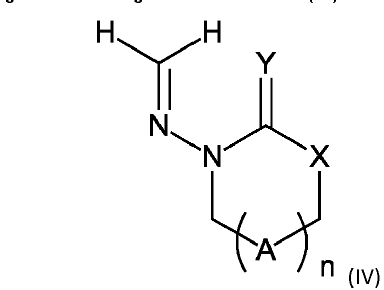 wobei Y, X, A und n wie in Formel (I) gemäß der vorliegenden Erfindung definiert sind, mit Aldehyden der Formel R¹-C(=O)H, Ketonen der Formel R¹-C(=O)-R² oder Mischungen davon, wobei R¹ und R² wie in Formel (I) definiert sind.

Schließlich betrifft die vorliegende Erfindung in einem sechsten Aspekt ein Verfahren zur langanhaltenden Beduftung von Oberflächen, dadurch gekennzeichnet, dass eine Duftvorläuferverbindung der vorliegenden Erfindung oder ein Wasch-, Reinigungsmittel oder ein kosmetisches Mittel der vorliegenden Erfindung auf die zu beduftende Oberfläche aufgebracht wird, wobei die Beduftung länger anhält als würde die jeweilige Duftstoffverbindung oder ein identisches Mittel in dem die Duftvorläuferverbindung durch die jeweilige Duftstoffverbindung ersetzt ist eingesetzt werden.

Diese und weitere Ausführungsformen, Merkmale und Vorteile der Erfindung werden für den Fachmann aus dem Studium der folgenden detaillierten Beschreibung und Ansprüche ersichtlich. Dabei können einzelne beschriebene Merkmale oder Ausführungsformen der Erfindung mit anderen Merkmalen oder Ausführungsformen der Erfindung kombiniert werden ohne dass diese im Rahmen der Erfindung in Kombination beschrieben wurden. Es ist selbstverständlich, dass die hierin enthaltenen Beispiele die Erfindung beschreiben und veranschaulichen sollen, diese aber nicht einschränken und insbesondere die Erfindung nicht auf die Beispiele beschränkt ist.

"Mindestens ein", wie hierin verwendet, bezieht sich auf 1 oder mehr, beispielsweise 2, 3, 4, 5, 6, 7, 8, 9 oder mehr. Im Zusammenhang mit den hierin beschriebenen Duftvorläuferverbindungen bezieht sich diese Angabe nicht auf die absolute Menge an Molekülen, sondern auf die Art der Verbindung. "mindestens eine Duftvorläuferverbindung" bedeutet daher beispielsweise, dass nur eine Art von Duftvorläuferverbindung oder mehrere verschiedene Arten von Duftvorläuferverbindungen, ohne Angaben über die Menge der einzelnen Verbindungen zu machen, enthalten sein können.

Alle im Zusammenhang mit dem hierin beschriebenen Verfahren angegeben Mengenangaben beziehen sich, sofern nichts anderes angegeben ist, auf Gew.-% jeweils bezogen auf das Gesamtgewicht der Zusammensetzung. Des Weiteren beziehen sich derartige Mengenangaben, die sich auf mindestens einen Bestandteil beziehen, immer auf die Gesamtmenge dieser Art von Bestandteil, der in der Zusammensetzung enthalten ist, sofern nicht explizit etwas anderes angegeben ist. Das heißt, dass sich derartige Mengenangaben, beispielsweise im Zusammenhang mit "mindestens einer Duftvorläuferverbindung", auf die Gesamtmenge von Duftvorläuferverbindungen, welche in der Zusammensetzung enthalten sind, bezieht, wenn nicht explizit etwas anderes angegeben ist.

Zahlenwerte, die hierin ohne Dezimalstellen angegeben sind, beziehen sich jeweils auf den vollen angegebenen Wert mit einer Dezimalstelle. So steht beispielsweise "99 %" für "99,0 %".

Numerische Bereiche, die in dem Format "in/von x bis y" angegeben sind, schließen die genannten Werte ein. Wenn mehrere bevorzugte numerische Bereiche in diesem Format angegeben sind, ist es selbstverständlich, dass alle Bereiche, die durch die Kombination der verschiedenen Endpunkte entstehen, ebenfalls erfasst werden.

"Substituiert", wie hierin im Zusammenhang mit der Definition der Duftstoffvorläuferverbindungen der Formeln (I) bis (III) verwendet, bedeutet, dass ein Wasserstoffatom durch einen anderen Rest ersetzt ist. Geeignete Reste schließen ein, sind aber nicht beschränkt auf, lineare oder verzweigte Kohlenwasserstoffgruppen mit bis zu 22 Kohlenstoffatomen, bevorzugt Kohlenwasserstoffgruppen mit bis zu 10 Kohlenstoffatomen, einschließlich Alkyl, Alkenyl, Alkinyl; -OH, -CN, -NO₂, -C(O)H, -C(O)OR', -C(O)NR'R", -NR'-C(O)-R", -NR'R", wobei R' und R" lineares oder verzweigtes Alkyl mit bis zu 12 Kohlenstoffatomen, bevorzugt lineares oder verzweigtes Alkyl mit bis zu 6 Kohlenstoffatomen, sind.

Die Begriffe "Riechstoff" und "Duftstoff" sind im Rahmen dieser Erfindung synonym zu verwenden. Ein Riechstoff ist eine Verbindung, die einen charakteristischen Geruch aufweist und zur Erzielung eines spezifischen Duftprofils eines Parfümöls oder einer Zusammensetzung beiträgt. Zu Riechstoffen gehören auch solche Verbindungen, die das Duftprofil eines Parfümöls oder einer Zusammensetzung dahingehend verändern, dass der Duft eine gewisse Tiefe erhält, was der Fachmann üblicherweise als Komplexität eines Duftes kennt.

Die vorliegende Erfindung betrifft insbesondere Duftvorläuferverbindungen der Formeln (I).

Eine Duftvorläuferverbindung der Formel (I) wobei 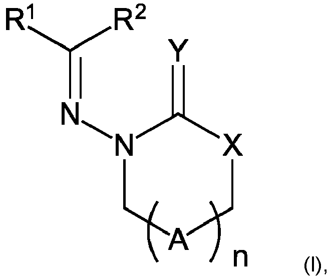
iii) R¹ und R² unabhängig voneinander ausgewählt sind aus H, oder linearen, verzweigten oder cyclischen, gesättigten oder ungesättigten, substituierten oder unsubstituierten Kohlenwasserstoffresten, die bis zu 20 Kohlenstoffatome und gegebenenfalls bis zu 6 Heteroatome enthalten;
   oder
iv) R¹ und R² gemeinsam mit dem Kohlenstoffatom an das sie gebunden sind einen cyclischen, gesättigten oder ungesättigten, Kohlenwasserstoffring mit bis zu 12 Kohlenstoffatomen bilden, der bis zu 6 Heteroatome enthalten kann;
   X und Y unabhängig voneinander ausgewählt sind aus O, S, Se oder NR³, wobei R³ H oder ein linearer oder verzweigter, gesättigter oder ungesättigter, substituierter oder unsubstituierter Kohlenwasserstoffrest mit bis zu 20 Kohlenstoffatomen ist, der gegebenenfalls bis zu 6 Heteroatome enthalten kann, bevorzugt aus O oder S;
   A gleich -CH₂-, -CHR⁴-, -CR⁴R⁵- oder ein Heteroatom ist, wobei R⁴ und R⁵ unabhängig voneinander ausgewählt sind aus linearen oder verzweigten, gesättigten oder ungesättigten, substituierten oder unsubstituierten Kohlenwasserstoffresten, die bis zu 20 Kohlenstoffatome und gegebenenfalls bis zu 6 Heteroatome enthalten, wobei falls mehrere Einheiten A vorhanden sind, diese unabhängig voneinander ausgewählt werden, bevorzugt ist A gleich -CH₂-, -CHR⁴- oder -CR⁴R⁵-, insbesondere-CH₂-;
   n = 0 bis 4, bevorzugt 0 oder 1, ist,
   mit der Maßgabe, dass sich der Rest R¹-C-R² von einem Duftstoffaldehyd oder einem Duftstoffketon der Formel R¹-C(=O)-R² ableitet, bevorzugt einem Duftstoffketon.

In einer Ausführungsform ist die mindestens eine Duftvorläuferverbindung der Formel (I) mindestens eine Duftvorläuferverbindung der Formel (II) wobei 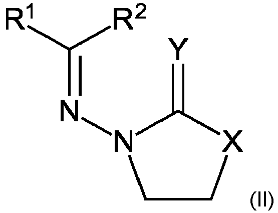
i) R¹ und R² unabhängig voneinander ausgewählt sind aus H, oder linearen, verzweigten oder cyclischen, gesättigten oder ungesättigten, substituierten oder unsubstituierten Kohlenwasserstoffresten, die bis zu 20 Kohlenstoffatome und gegebenenfalls bis zu 6 Heteroatome enthalten;
   oder
ii) R¹ und R² gemeinsam mit dem Kohlenstoffatom an das sie gebunden sind einen cyclischen, gesättigten oder ungesättigten, Kohlenwasserstoffring mit bis zu 12 Kohlenstoffatomen bilden, der bis zu 6 Heteroatome enthalten kann;
   X und Y unabhängig voneinander ausgewählt sind aus O, S, Se oder NR³, wobei R³ H oder ein linearer oder verzweigter, gesättigter oder ungesättigter, substituierter oder unsubstituierter Kohlenwasserstoffrest mit bis zu 20 Kohlenstoffatomen ist, der gegebenenfalls bis zu 6 Heteroatome enthalten kann, bevorzugt aus O oder S;
   mit der Maßgabe, dass sich der Rest R¹-C-R² von einem Duftstoffaldehyd oder einem Duftstoffketon der Formel R¹-C(=O)-R² ableitet, bevorzugt einem Duftstoffketon.

In einer weiteren Ausführungsform ist die mindestens eine Duftvorläuferverbindung der Formel (I) mindestens eine Duftvorläuferverbindung der Formel (III) 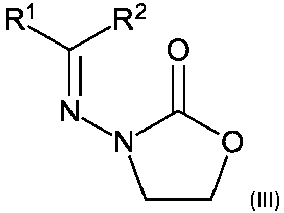 wobei
i) R¹ und R² unabhängig voneinander ausgewählt sind aus H, oder linearen, verzweigten oder cyclischen, gesättigten oder ungesättigten, substituierten oder unsubstituierten Kohlenwasserstoffresten, die bis zu 20 Kohlenstoffatome und gegebenenfalls bis zu 6 Heteroatome enthalten;
   oder
ii) R¹ und R² gemeinsam mit dem Kohlenstoffatom an das sie gebunden sind einen cyclischen, gesättigten oder ungesättigten, Kohlenwasserstoffring mit bis zu 12 Kohlenstoffatomen bilden, der bis zu 6 Heteroatome enthalten kann;
   mit der Maßgabe, dass sich der Rest R¹-C-R² von einem Duftstoffaldehyd oder einem Duftstoffketon der Formel R¹-C(=O)-R² ableitet, bevorzugt von einem Duftstoffketon.

Aus den beschriebenen Duftvorläuferverbindungen der Formel (I) bis (III) kann der Duftstoff durch Hydrolyse freigesetzt werden, insbesondere im Sauren. Die Duftvorläuferverbindungen der vorliegenden Erfindung werden dabei durch Umsetzen eines entsprechenden Hydrazids mit dem Duftstoff, hier ein Duftstoffaldehyd oder-keton, insbesondere ein Duftstoffketon, erhalten.

In verschiedenen Ausführungsformen der Erfindung kann das Duftstoff-Aldehyd ausgewählt werden aus Adoxal (2,6,10-Trimethyl-9-undecenal), Anisaldehyd (4-Methoxybenzaldehyd), Cymal oder Cyclamenaldehyd (3-(4-Isopropylphenyl)-2-methylpropanal), Nympheal (3-(4-isobutyl-2-methylphenyl)propanal), Ethylvanillin, Florhydral (3-(3-isopropylphenyl)butanal]), Trifernal (3-Phenylbutyraldehyd), Helional (3-(3,4-Methylendioxyphenyl)-2-methylpropanal), Heliotropin, Hydroxycitronellal, Lauraldehyd, Lyral (3- und 4-(4-Hydroxy-4-methylpentyl)-3-cyclohexen-1-carboxaldehyd), Methylnonylacetaldehyd, Lilial (3-(4-tert-Butylphenyl)-2-methylpropanal), Phenylacetaldehyd, Undecylenaldehyd, Vanillin, 2,6,10-Trimethyl-9-undecenal, 3-Dodecen-1-al, alpha-n-Amylzimtaldehyd, Melonal (2,6-Dimethyl-5-heptenal), Triplal (2,4-Dimethyl-3-cyclohexen-1-carboxaldehyd), 4-Methoxybenzaldehyd, Benzaldehyd, 3-(4-tert-Butylphenyl)-propanal, 2-Methyl-3-(para-methoxyphenyl)propanal, 2-Methyl-4-(2,6,6-timethyl-2(1)-cyclohexen-1-yl)butanal, 3-Phenyl-2-propenal, cis-/trans-3,7-Dimethyl-2,6-octadien-1-al, 3,7-Dimethyl-6-octen-1-al, [(3,7-Dimethyl-6-octenyl)oxy]acetaldehyd, 4-Isopropylbenzylaldehyd, 1,2,3,4,5,6,7,8-Octahydro-8,8-dimethyl-2-naphthaldehyd, 2,4-Dimethyl-3-cyclohexen-1-carboxaldehyd, 2-Methyl-3-(isopropylphenyl)propanal, 1-Decanal, 2,6-Dimethyl-5-heptenal, 4-(Tricyclo[5.2.1.0(2,6)]-decyliden-8)-butanal, Octahydro-4,7-methan-1H-indencarboxaldehyd, 3-Ethoxy-4-hydroxybenzaldehyd, para-Ethyl-alpha,alpha-dimethylhydrozimtaldehyd, alpha-Methyl-3,4-(methylendioxy)-hydrozimtaldehyd, 3,4-Methylendioxybenzaldehyd, alpha-n-Hexylzimtaldehyd, m-Cymen-7-carboxaldehyd, alpha-Methylphenylacetaldehyd, Tetrahydrocitral (3,7-Dimethyloctanal), Undecenal, 2,4,6-Trimethyl-3-cyclohexen-1-carboxaldehyd, 4-(3)(4-Methyl-3-pentenyl)-3-cyclohexencarboxaldehyd, 1-Dodecanal, 2,4-Dimethylcyclohexen-3-carboxaldehyd, 4-(4-Hydroxy-4-methylpentyl)-3-cylohexen-1-carboxaldehyd, 7-Methoxy-3,7-dimethyloctan-1-al, 2-Methyldecanal, 1-Nonanal, 1-Octanal, 2,6,10-Trimethyl-5,9-undecadienal, 2-Methyl-3-(4-tert-butyl)propanal, Dihydrozimtaldehyd, 1-Methyl-4-(4-methyl-3-pentenyl)-3-cyclohexen-1-carboxaldehyd, 5- oder 6-Methoxyhexahydro-4,7-methanindan-1- oder -2-carboxaldehyd, 3,7-Dimethyloctan-1-al, 1-Undecanal, 10-Undecen-1-al, 4-Hydroxy-3-methoxybenzaldehyd, 1-Methyl-3-(4-methylpentyl)-3-cyclohexencarboxaldehyd, 7-Hydroxy-3,7-dimethyl-octanal, trans-4-Decenal, 2,6-Nonadienal, para-Tolylacetaldehyd, 4-Methylphenylacetaldehyd, 2-Methyl-4-(2,6,6-trimethyl-1-cyclohexen-1-yl)-2-butenal, ortho-Methoxyzimtaldehyd, 3,5,6-Trimethyl-3-cyclohexencarboxaldehyd, 3,7-Dimethyl-2-methylen-6-octenal, Phenoxyacetaldehyd, 5,9-Dimethyl-4,8-decadienal, Päonienaldehyd (6,10-Dimethyl-3-oxa-5,9-undecadien-1-al), Hexahydro-4,7-methanindan-1-carboxaldehyd, 2-Methyloctanal, alpha-Methyl-4-(1-methylethyl)benzolacetaldehyd, 6,6- Dimethyl-2-norpinen-2-propionaldehyd, para-Methylphenoxyacetaldehyd, 2-Methyl-3-phenyl-2-propen-1-al, 3,5,5-Trimethylhexanal, Hexahydro-8,8-dimethyl-2-naphthaldehyd, 3-Propylbicyclo[2.2.1]-hept-5-en-2-carbaldehyd, 9-Decenal, 3-Methyl-5-phenyl-1-pentanal, Floral (4,8-Dimethyl-4,9-decadienal), Aldehyd C12MNA (2-Methylundecanal), Liminal (beta-4-Dimethylcyclohex-3-ene-1-propan-1-al), Methylnonylacetaldehyd, Hexanal, trans-2-Hexenal und Mischungen davon.

Geeignete Ketone schließen ein, sind aber nicht beschränkt auf Methyl-beta-naphthylketon, Moschusindanon (1,2,3,5,6,7-hexahydro-1,1,2,3,3-pentamethyl-4H-inden-4-on), Calone (Methylbenzodioxepinon), Tonalid (6-Acetyl-1,1,2,4,4,7-hexamethyltetralin), alpha-Damascone, beta-Damascone, delta-Damascone, iso-Damascone, Damascenone, Methyldihydrojasmonat (Hedion), Menthon, Carvon, Kampfer, Koavon (3,4,5,6,6-pentamethylhept-3-en-2-on), Fenchon, alpha-Ionon, beta-Ionon, Dihydro-beta-Ionon, gamma-Methyl-lonon, Fleuramon (2-heptylcyclopentanon), Frambinonmethylether (4-(4-methoxyphenyl)butan-2-on), Dihydrojasmon, cis-Jasmon, 1-(1,2,3,4,5,6,7,8-octahydro-2,3,8,8-tetramethyl-2-naphthalenyl)-ethan-1-on und Isomere davon, Methylcedrenylketon, Acetophenon, Methylacetophenon, para-Methoxyacetophenon, Methyl-betanaphtylketon, Benzylaceton, Benzophenon, para-Hydroxyphenylbutanon, Sellerie-Keton(3- methyl-5-propyl-2-cyclohexenon), 6-Isopropyldeca-hydro-2-naphton, Dimethyloctenon, Frescomenthe (2-butan-2-ylcyclohexan-1-on), 4-(1-Ethoxyvinyl)-3,3,5,5-tetramethylcyclohexanon, Methylheptenon, 2-(2-(4-Methyl-3-cyclohexen-1-yl)propyl)cyclopen-tanon, 1-(p-Menthen-6(2)yl)-1-propanon, 4-(4-Hydroxy-3-methoxyphenyl)-2-butanon, 2-Acetyl-3,3-dimethylnorbornan, 6,7-Dihydro-1,1,2,3,3-pentamethyl-4(5H)in-danon, 4-Damascol, Dulcinyl (4-(1,3-benzodioxol-5-yl)butan-2-on), Hexalon (1-(2,6,6-trimethyl-2-cyclohexene-1-yl)-1,6-heptadien-3-on), Isocyclemon E (2-acetonaphthon-1 ,2,3,4,5,6,7,8-octahydro-2,3,8,8-tetramethyl), Methylnonylketon, Methylcyclocitron, Methyllavendelketon, Orivon (4-tert-amylcyclohexanon), 4-tert-butyl cyclohexanon, Delphon (2-pentyl cyclopentanon), Muscon (CAS 541 - 91 -3), Neobutenon (1-(5,5-dimethyl-1-cyclo-hexenyl)pent-4-en-1-on), Plicaton (CAS 41724-19-0), Velouton (2,2,5-trimethyl-5-pentylcyclopentan-1-on), 2,4,4,7-Tetramethyl-oct-6-en-3-on, Tetrameran (6,10-dimethylundecen-2-on) und Mischungen davon.

Bevorzugt ist das Duftstoffaldehyd oder -keton ausgewählt aus Dihydro-beta-ionon, Frambinonmethylether, Hedion, Benzylaceton, Calone, Decanal, Cyclamenaldehyd, Melonal und Anisaldehyd, insbesondere aus Dihydroionon, Frambinonmethylether, Hedion, Benzylaceton, Calone, Decanal, Cyclamenaldehyd, Melonal. In einer Ausführungsform der Erfindung ist Anisaldehyd nicht enthalten.

Darüber hinaus können als Duftstoff-Aldehyde und/oder Duftstoff-Ketone grundsätzlich alle üblichen Duftstoff-Aldehyde und/oder Duftstoff-Ketone, die nach Umsetzung unter die Definition der oben genannten Formel (I) fallen, eingesetzt werden, die insbesondere zur Herbeiführung eines angenehmen Geruchsempfindens beim Menschen eingesetzt werden. Solche Duftstoff-Aldehyde und/oder Duftstoff-Ketone sind dem Fachmann bekannt und auch in der Patentliteratur, beispielsweise in US 2003/0158079 A1, Absätze [0154] und [0155] beschrieben. Für weitere geeignete Duftstoffe sei auf Steffen Arctander, Aroma Chemicals Band 1 und Band 2 (veröffentlicht 1960 bzw. 1969, Neuauflage 2000; ISBN: 0-931710-37-5 und 0-931710-38-3) verwiesen.

Zur Herstellung der Duftvorläuferverbindung kann eine Verbindung der allgemeinen Formel (IV) 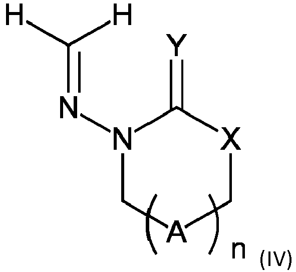 wobei Y, X, A und n wie in Formel (I) definiert sind, mit Aldehyden, Ketonen oder Mischungen von Ketonen und Aldehyden umgesetzt werden, die nach Umsetzung mit dem C Atom an das sie gebunden sind den Rest R¹-C-R² wie definiert in Formel (I) bilden. Folglich handelt es sich dabei um Aldehyde der Formel R¹-C(=O)H und Ketone der Formel R¹-C(=O)-R², wobei R¹ und R² wie in Formel (I) definiert sind.

Die Umsetzung findet dabei bevorzugt unter Stickstoffatmosphäre statt. Ferner wird die Umsetzung bevorzugt in einem geeigneten Lösungsmittel durchgeführt. Geeignete Lösungsmittel sind beispielsweise aromatenhaltige Kohlenwasserstoffe wie Toluol. Die Umsetzung wird dabei bevorzugt bei einer Temperatur im Bereich von 80 bis 150 °C, besonders bevorzugt 100 bis 140 °C durchgeführt. Beispielsweise wird das Hydrazid mit der oben dargestellten allgemeinen Formel unter Stickstoffatmosphäre zusammen mit dem gewünschten Keton und/oder Aldehyd im Lösungsmittel vorgelegt. Sodann wird das Reaktionsgemisch erhitzt. Häufig wird sodann unter Rückfluss am Wasserabscheider erhitzt. Das erhaltene Umsetzungsprodukt wird nach üblichen Verfahren isoliert und gegebenenfalls gereinigt.

Die oben beschriebenen Duftvorläuferverbindungen der Formel (I) bis (III) können in den Mitteln des erfindungsgemäßen Verfahrens als Mischungen mit mindestens einem weiteren Duftstoff oder mindestens einer weiteren Duftvorläuferverbindung, welche von der Duftvorläuferverbindung nach Formel (I) verschieden ist, eingesetzt werden.

Die weiteren Duftstoffe, die in den Zusammensetzungen optional enthalten sein können, sind keinen besonderen Beschränkungen unterworfen. So können einzelne Duftstoffverbindungen natürlichen oder synthetischen Ursprungs, z.B. vom Typ der Ester, Ether, Aldehyde, Ketone, Alkohole und Kohlenwasserstoffe, verwendet werden. Duftstoffverbindungen vom Typ der Ester sind z.B. Benzylacetat, Phenoxyethylisobutyrat, p-tert-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmacyclat. Zu den Ethern zählen beispielsweise Benzylethylether und Ambroxan, zu den Aldehyden die oben genannten z.B. die linearen Alkanale mit 8 bis 18 C-Atomen, Citral, Citronellal, Citronellyloxyacetaldehyd, Cyclamenaldehyd (3-(4-propan-2-ylphenyl)butanal), Lilial und Bourgeonal, zu den Ketonen z.B. die Ionone, [alpha]-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich Terpene wie Limonen und Pinen. Bevorzugt werden jedoch Mischungen verschiedener Duftstoffe verwendet, die gemeinsam eine ansprechende Duftnote erzeugen.

Die Zusammensetzungen können ferner auch natürliche Duftstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller-Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliöl, Orangenschalenöl und Sandelholzöl. Weitere herkömmliche Duftstoffe, die im Rahmen der vorliegenden Erfindung in den erfindungsgemäßen Mitteln enthalten sein können, sind beispielsweise die ätherischen Öle wie Angelikawurzelöl, Anisöl, Arnikablütenöl, Basilikumöl, Bayöl, Champacablütenöl, Edeltannenöl, Edeltannenzapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Copaivabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Origanumöl, Palmarosaöl, Patchuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wntergrünöl, Ylang-Ylang-Öl, Ysop-Öl, Zimtöl, Zimtblätteröl, Zitronellöl, Zitronenöl sowie Zypressenöl sowie Ambrettolid, Ambroxan, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, Boisambrene forte, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iran, Isoeugenol, Isoeugenolmethyl-ether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphthol-methylether, Nerol, n-Nonylaldehyd, Nonylalkohol, n-Octyl-aldehyd, p-Oxy-Acetophenon, Pentadekanolid, β-Phenylethylalkohol, Phenylessigsäure, Pulegon, Safrol, Salicylsäureisoamylester, Salicylsäuremethylester, Salicylsäurehexylester, Salicylsäurecyclohexylester, Santalol, Sandelice, Skatol, Terpineol, Thymen, Thymol, Troenan, γ-Undelacton, Vanillin, Veratrumaldehyd, Zimtaldehyd, Zimatalkohol, Zimtsäure, Zimtsäureethylester, Zimtsäurebenzylester, Diphenyloxid, Limonen, Linalool, Linalylacetat und -propionat, Melusat, Menthol, Menthon, Methyl-n-heptenon, Pinen, Phenylacetaldehyd, Terpinylacetat, Citral, Citronellal und Mischungen daraus.

Es ist außerdem möglich, dass die mindestens eine Duftvorläuferverbindungen der Formel (I) bis Formel (III) mit den korrespondierenden Aldehyden und/oder Ketonen eingesetzt werden. Gemäß einer bevorzugten Ausführungsform des erfindungsgemäßen Verfahrens zeichnen sich derartige Zusammensetzungen dadurch aus, dass das Molverhältnis von Duftstoff-Aldehyd und/oder DuftstoffKeton zu der korrespondierenden Duftvorläuferverbindung der Formel (I) bis (III) 20:1 bis 1:20, bevorzugt 10:1 bis 1:10, stärker bevorzugt 5:1 bis 1:5, noch stärker bevorzugt 3:1 bis 1:3, noch stärker bevorzugt 2:1 bis 1:2 und insbesondere 1,2:1 bis 1:1,2 beträgt.

Die vorliegende Erfindung betrifft ferner Wasch-, Reinigungs-, Luftpflege- oder kosmetische Mittel, welche mindestens eine der Duftvorläuferverbindungen der vorliegenden Erfindung enthalten. In bevorzugten Ausführungsformen ist die mindestens eine Duftvorläuferverbindung in einer Gesamtmenge von 0,001 und 5 Gew.-%, vorteilhafterweise von 0,005 und 4 Gew.-%, weiter vorteilhaft von 0,01 und 2 Gew.-%, jeweils bezogen auf das Gesamtgewicht des jeweiligen Mittels, enthalten.

Die im Verfahren einzusetzenden Wasch- und Reinigungsmittel können ferner anionische, nichtionische, kationische, amphotere oder zwitterionische Tenside oder Mischungen davon enthalten. Ferner können diese Mittel in fester oder flüssiger Form vorliegen.

Geeignete nichtionische Tenside sind insbesondere Ethoxylierungs- und/oder Propoxylierungsprodukte von Alkylglykosiden und/oder linearen oder verzweigten Alkoholen mit jeweils 12 bis 18 C-Atomen im Alkylteil und 3 bis 20, vorzugsweise 4 bis 10 Alkylethergruppen. Weiterhin sind entsprechende Ethoxylierungs- und/oder Propoxylierungsprodukte von N-Alkylaminen, vicinalen Diolen, Fettsäureestern und Fettäureamiden, die hinsichtlich des Alkylteils den genannten langkettigen Alkoholderivaten entsprechen, sowie von Alkylphenolen mit 5 bis 12 C-Atomen im Alkylrest brauchbar.

Geeignete anionische Tenside sind insbesondere Seifen und solche, die Sulfat- oder Sulfonat-Gruppen mit bevorzugt Alkaliionen als Kationen enthalten. Verwendbare Seifen sind bevorzugt die Alkalisalze der gesättigten oder ungesättigten Fettsäuren mit 12 bis 18 C-Atomen. Derartige Fettsäuren können auch in nicht vollständig neutralisierter Form eingesetzt werden. Zu den brauchbaren Tensiden des Sulfat-Typs gehören die Salze der Schwefelsäurehalbester von Fettalkoholen mit 12 bis 18 C-Atomen und die Sulfatierungsprodukte der genannten nichtionischen Tenside mit niedrigem Ethoxylierungsgrad. Zu den verwendbaren Tensiden vom Sulfonat-Typ gehören lineare Alkylbenzolsulfonate mit 9 bis 14 C-Atomen im Alkylteil, Alkansulfonate mit 12 bis 18 C-Atomen, sowie Olefinsulfonate mit 12 bis 18 C-Atomen, die bei der Umsetzung entsprechender Monoolefine mit Schwefeltrioxid entstehen, sowie alpha-Sulfofettsäureester, die bei der Sulfonierung von Fettsäuremethyl- oder -ethylestern entstehen.

Kationische Tenside werden vorzugsweise unter den Esterquats und/oder den quaternären Ammoniumverbindungen (QAV) gemäß der allgemeinen Formel (R^{I})(R^{II})(R^{III})(R^{IV})N⁺ X⁻ ausgewählt, in der R^{I} bis R^{IV} für gleiche oder verschiedene C₁₋₂₂-Alkylreste, C₇₋₂₈-Arylalkylreste oder heterozyklische Reste stehen, wobei zwei oder im Falle einer aromatischen Einbindung wie im Pyridin sogar drei Reste gemeinsam mit dem Stickstoffatom den Heterozyklus, z.B. eine Pyridinium- oder Imidazoliniumverbindung, bilden, und X⁻ für Halogenidionen, Sulfationen, Hydroxidionen oder ähnliche Anionen steht. QAV sind durch Umsetzung tertiärer Amine mit Alkylierungsmitteln, wie z.B. Methylchlorid, Benzylchlorid, Dimethylsulfat, Dodecylbromid, aber auch Ethylenoxid herstellbar. Die Alkylierung von tertiären Aminen mit einem langen Alkyl-Rest und zwei Methyl-Gruppen gelingt besonders leicht, auch die Quaternierung von tertiären Aminen mit zwei langen Resten und einer Methyl-Gruppe kann mit Hilfe von Methylchlorid unter milden Bedingungen durchgeführt werden. Amine, die über drei lange Alkyl-Reste oder Hydroxy-substituierte Alkyl-Reste verfügen, sind wenig reaktiv und werden z.B. mit Dimethylsulfat quaterniert. In Frage kommende QAV sind beispielweise Benzalkoniumchlorid (N Alkyl-N,N dimethyl-benzylammoniumchlorid), Benzalkon B (m,p-Dichlorbenzyldimethyl-C₁₂-alkylammoniumchlorid, Benzoxoniumchlorid (Benzyl-dodecyl-bis-(2-hydroxyethyl)-ammoniumchlorid), Cetrimoniumbromid (N-Hexadecyl-N,N-trimethyl-ammoniumbromid), Benzetoniumchlorid (N,N Dimethyl-N [2-[2-[p-(1,1,3,3-tetramethylbutyl)phenoxy]-ethoxy]-ethyl]-benzylammoniumchlorid), Dialkyldimethylammoniumchloride wie Di-n-decyl-dimethyl-ammoniumchlorid, Didecyldimethylammoniumbromid, Dioctyl-dimethyl-ammoniumchlorid, 1-Cetylpyridiniumchlorid und Thiazolinjodid sowie deren Mischungen. Bevorzugte QAV sind die Benzalkoniumchloride mit C₈-C₂₂-Alkylresten, insbesondere C₁₂-C₁₄-Alkylbenzyl-dimethylammoniumchlorid.

Bevorzugte Esterquats sind Methyl-N-(2-hydroxyethyl)-N,N-di(talgacyl-oxyethyl)ammonium-methosulfat, Bis-(palmitoyl)-ethyl-hydroxyethyl-methyl-ammonium-methosulfat oder Methyl-N,N-bis(acyloxyethyl)-N-(2-hydroxyethyl)ammonium-methosulfat. Handelsübliche Beispiele sind die von der Firma Stepan unter dem Warenzeichen Stepantex® vertriebenen Methylhydroxyalkyldialkoyloxyalkylammoniummethosulfate oder die unter dem Handelsnamen Dehyquart® bekannten Produkte der Firma BASF SE beziehungsweise die unter der Bezeichnung Rewoquat® bekannten Produkte des Herstellers Evonik.

Ferner können die Wasch- und Reinigungsmittel weitere Inhaltsstoffe enthalten, die die anwendungstechnischen und/oder ästhetischen Eigenschaften der Zusammensetzung abhängig von dem beabsichtigten Verwendungszweck weiter verbessern. Im Rahmen der vorliegenden Erfindung können sie Builder, Bleichmittel, Bleichaktivatoren, Bleichkatalysatoren, Esterquats, Silikonöle, Emulgatoren, Verdicker, Elektrolyte, pH-Stellmittel, Fluoreszenzmittel, Farbstoffe, Hydrotope, Schauminhibitoren, Antiredepositionsmittel, Lösungsmittel, Enzyme, optischen Aufheller, Vergrauungsinhibitoren, Einlaufverhinderer, Knitterschutzmittel, Farbübertragungsinhibitoren, Farbschutzmittel, Benetzungsverbesserer, antimikrobiellen Wirkstoffen, Germizide, Fungizide, Antioxidantien, Korrosionsinhibitoren, Klarspüler, Konservierungsmittel, Antistatika, Bügelhilfsmittel, Phobier- und Imprägniermittel, Perlglanzmittel, Polymere, Quell- und Schiebefestmittel sowie UV-Absorber enthalten, ohne darauf beschränkt zu sein.

Die Mengen der einzelnen Inhaltsstoffe in den Wasch- und Reinigungsmitteln orientieren sich jeweils am Einsatzzweck der betreffenden Zusammensetzung und der Fachmann ist mit den Größenordnungen der einzusetzenden Mengen der Inhaltsstoffe grundsätzlich vertraut oder kann diese der zugehörigen Fachliteratur entnehmen. Je nach Einsatzzweck der Zusammensetzungen wird man beispielsweise den Tensidgehalt höher oder niedriger wählen. Üblicherweise kann z.B. der Tensidgehalt beispielsweise von Waschmitteln von 10 bis 50 Gew.-%, bevorzugt von 12,5 bis 30 Gew.-% und stärker bevorzugt von 15 bis 25 Gew.-% betragen.

Die Wasch- und Reinigungsmittel können beispielsweise mindestens einen wasserlöslichen und/oder wasserunlöslichen, organischen und/oder anorganischen Builder enthalten. Zu den wasserlöslichen organischen Buildersubstanzen gehören Polycarbonsäuren, insbesondere Citronensäure und Zuckersäuren, monomere und polymere Aminopolycarbonsäuren, insbesondere Methylglycindiessigsäure, Nitrilotriessigsäure und Ethylendiamintetraessigsäure sowie Polyasparaginsäure, Polyphosphonsäuren, insbesondere Aminotris(methylenphosphonsäure), Ethylendiamintetrakis(methylenphosphonsäure) und 1-Hydroxyethan-1,1-diphosphonsäure, polymere Hydroxyverbindungen wie Dextrin sowie polymere (Poly-)carbonsäuren, polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Geeignete, wenn auch weniger bevorzugte Verbindungen dieser Klasse sind Copolymere der Acrylsäure oder Methacrylsäure mit Vinylethern, wie Vinylmethylethern, Vinylester, Ethylen, Propylen und Styrol, in denen der Anteil der Säure mindestens 50 Gew.-% beträgt. Die organischen Buildersubstanzen können, insbesondere zur Herstellung flüssiger Wasch- und Reinigungsmittel, in Form wässriger Lösungen, vorzugsweise in Form 30- bis 50-gewichtsprozentiger wässriger Lösungen eingesetzt werden. Alle genannten Säuren werden in der Regel in Form ihrer wasserlöslichen Salze, insbesondere ihre Alkalisalze, eingesetzt.

Organische Buildersubstanzen können, falls gewünscht, in Mengen bis zu 40 Gew.-%, insbesondere bis zu 25 Gew.-% und vorzugsweise von 1 Gew.-% bis 8 Gew.-% enthalten sein. Mengen nahe der genannten Obergrenze werden vorzugsweise in pastenförmigen oder flüssigen, insbesondere wasserhaltigen, erfindungsgemäßen Mitteln eingesetzt. Wäschenachbehandlungsmittel, wie z.B. Weichspüler, können gegebenenfalls auch frei von organischem Builder sein.

Als wasserlösliche anorganische Buildermaterialien kommen insbesondere Alkalisilikate und Polyphosphate, vorzugsweise Natriumtriphosphat, in Betracht. Als wasserunlösliche, wasserdispergierbare anorganische Buildermaterialien können insbesondere kristalline oder amorphe Alkalialumosilikate, falls gewünscht, in Mengen von bis zu 50 Gew.-%, vorzugsweise nicht über 40 Gew.-% und in flüssigen Zusammensetzungen insbesondere von 1 Gew.-% bis 5 Gew.-%, eingesetzt werden. Unter diesen sind die kristallinen Natriumalumosilikate in Waschmittelqualität, insbesondere Zeolith A, P und gegebenenfalls X, bevorzugt. Mengen nahe der genannten Obergrenze werden vorzugsweise in festen, teilchenförmigen Mitteln eingesetzt. Geeignete Alumosilikate weisen insbesondere keine Teilchen mit einer Korngröße über 30 µm auf und bestehen vorzugsweise zu wenigstens 80 Gew.-% aus Teilchen mit einer Größe unter 10 µm.

Geeignete Substitute beziehungsweise Teilsubstitute für das genannte Alumosilikat sind kristalline Alkalisilikate, die allein oder im Gemisch mit amorphen Silikaten vorliegen können. Die in Wasch- oder Reinigungsmitteln als Gerüststoffe brauchbaren Alkalisilikate weisen vorzugsweise ein molares Verhältnis von Alkalioxid zu SiO₂ unter 0,95, insbesondere von 1:1,1 bis 1:12 auf und können amorph oder kristallin vorliegen. Bevorzugte Alkalisilikate sind die Natriumsilikate, insbesondere die amorphen Natriumsilikate, mit einem molaren Verhältnis Na₂O:SiO₂ von 1:2 bis 1:2.8. Als kristalline Silikate, die allein oder im Gemisch mit amorphen Silikaten vorliegen können, werden vorzugsweise kristalline Schichtsilikate der allgemeinen Formel Na₂SiₓO₂ₓ₊₁·yH₂O eingesetzt, in der x, das sogenannte Modul, eine Zahl von 1,9 bis 4 und y eine Zahl von 0 bis 20 ist und bevorzugte Werte für x 2, 3 oder 4 sind. Bevorzugte kristalline Schichtsilikate sind solche, bei denen x in der genannten allgemeinen Formel die Werte 2 oder 3 annimmt. Insbesondere sind sowohl beta- als auch delta-Natriumdisilikate (Na₂Si₂O₅·yH₂O) bevorzugt. Auch aus amorphen Alkalisilikaten hergestellte, praktisch wasserfreie kristalline Alkalisilikate der obengenannten allgemeinen Formel, in der x eine Zahl von 1,9 bis 2,1 bedeutet, können eingesetzt werden. In einer weiteren bevorzugten Ausführungsform wird ein kristallines Natriumschichtsilikat mit einem Modul von 2 bis 3 eingesetzt, wie es aus Sand und Soda hergestellt werden kann. Kristalline Natriumsilikate mit einem Modul im Bereich von 1,9 bis 3,5 werden in einer weiteren bevorzugten Ausführungsform der Textilbehandlungs- oder Reinigungsmittel eingesetzt. Falls als zusätzliche Buildersubstanz auch Alkalialumosilikat, insbesondere Zeolith, vorhanden ist, beträgt das Gewichtsverhältnis Alumosilikat zu Silikat, jeweils bezogen auf wasserfreie Aktivsubstanzen, vorzugsweise 1:10 bis 10:1. In Zusammensetzungen, die sowohl amorphe als auch kristalline Alkalisilikate enthalten, beträgt das Gewichtsverhältnis von amorphem Alkalisilikat zu kristallinem Alkalisilikat vorzugsweise 1:2 bis 2:1 und insbesondere 1:1 bis 2:1.

Buildersubstanzen sind, falls gewünscht, bevorzugt in Mengen bis zu 60 Gew.-%, insbesondere von 5 Gew.-% bis 40 Gew.-%, enthalten. Wäschenachbehandlungsmittel, wie z.B. Weichspüler, sind bevorzugt frei von anorganischem Builder.

Geeignete Inhaltsstoffe und Rahmenzusammensetzungen für Wasch- und Reinigungsmittelzusammensetzungen (beispielsweise für Waschmittel und Weichspüler) sind beispielsweise in der EP 3 110 393 B1 offenbart.

Geeignete Inhaltsstoffe und Rahmenzusammensetzungen für kosmetische Mittel, wie Haarpflegemittelzusammensetzungen, sind beispielsweise in der DE 102017215071 A1 offenbart.

### BEISPIELE

### Herstellungsbeispiel 1: 3-Amino-1,3-oxazolidin-2-on (A)

Ein 250 mL-Schlenkkolben wurde sekuriert und ausgeheizt. Unter Stickstoffatmosphäre wurden 1.74 g 2-Oxazolidinon in 50 mL trockenem 1,4-Dioxan vorgelegt. Bei Raumtemperatur wurden 0.96 g Natriumhydrid hinzugefügt. Das Gemisch wurde 1 h bei 60°C umgesetzt. Anschließend wurde auf Raumtemperatur abgekühlt. Durch ein Eisbad gekühlt, wurden nun 2.38 g Hydroxylamin-O-sulfonsäure portionsweise hinzugefügt. Das Eisbad wurde entfernt und die Reaktion 24 h bei Raumtemperatur gerührt. Das Gemisch wurde durch ein Eisbad gekühlt und mit Ethanol und destilliertem Wasser versetzt. Das Rohprodukt wurde über Celite filtriert und das Lösungsmittel des Filtrats unter vermindertem Druck entfernt. Das Produkt wurde in Wasser gelöst, die Lösung mit Methanol überschichtet und im Kühlschrank gelagert. Das gewünschte Produkt A wurde in einem Gemisch mit nicht abreagiertem 2-Oxazolidinon erhalten (m = 1.50 g).

### Beispiel 2: 3-{[4-(2,6,6-trimethylcyclohex-2-en-1-yl)butan-2-yliden]amino}-1,3-oxazolidin-2-on (B)

In einen 250 mL-Einhalskolben wurden 0.60 g 3-Amino-1,3-oxazolidin-2-on in 50 mL Toluol vorgelegt. 2.50 g Dihydro-beta-ionon und 0.04 g p-Toluolsulfonsäure Monohydrat wurden hinzugefügt und das Gemisch bei 125 °C bis zum vollständigen Umsatz (3 h) gerührt. Anschließend wurde auf Raumtemperatur abgekühlt, das Lösungsmittel unter vermindertem Druck entfernt und säulenchromatographisch (Laufmittel Hexan:Ethylacetat) aufgereinigt. Das gewünschte Produkt **B** wurde als gelbliches Öl erhalten (m = 0.85 g, 48% über zwei Stufen). R_{f} = 0.49 (Hexan: Ethylacetat; 2:1). FTIR (film): ṽ = 2926, 1755, 1638, 1477, 1382, 1208, 1033, 755 cm⁻¹. ¹H-NMR (400 MHz, DMSO-d₆): δ = 4.35 (t, *J* = 7.7 Hz, 2H), 3.75 (t, *J* = 7.7 Hz, 2H), 2.40 - 2.45 (m, 2 H), 2.18 - 2.21 (m, 2H), 2.00 - 2.02 (m, 1H), 1.95 (s, 3H), 1.85 - 1.89 (m, 1H), 1.58 (s, 3H), 1.50 - 1.52 (m, 2H), 1.35 - 1.41 (m, 2H), 1.00 (s, 6H) ppm. ¹³C-NMR (125.8 MHz, DMSO-d₆): δ = 174.7 (C_{quart}), 155.4 (C_{quart}), 136.3 (C_{quart}), 127.6 (C_{quart}), 62.1 (CH₂), 48.5 (CH₂), 39.1 (CH₂), 35.0 (CH), 32.6 (CH₂), 29.9 (2 CH₃), 28.8 (CH₂), 28.5 (CH), 24.8 (CH₃), 21.1 (CH₂), 19.4 (CH₃) ppm. MS (ESI, positive ion): 279 ([M+H]⁺, 100%).

### Beispiel 3: 3-{[4-(4-Methoxyphenyl)butan-2-ylidene]amino}-1,3-oxazolidin-2-on (C)

In einen 250 mL-Einhalskolben wurden 0.70 g 3-Amino-1,3-oxazolidin-2-on in 50 mL Toluol vorgelegt. 2.70 g Frambinonmethylether und 0.04 g p-Toluolsulfonsäure Monohydrat wurden hinzugefügt und das Gemisch bei 125 °C bis zum vollständigen Umsatz (2 h) gerührt. Anschließend wurde auf Raumtemperatur abgekühlt, das Lösungsmittel unter vermindertem Druck entfernt und säulenchromatographisch (Laufmittel Hexan:Ethylacetat) aufgereinigt. Das gewünschte Produkt **(C)** wurde als gelbliches Öl erhalten (m = 0.85 g, 64 % über zwei Stufen). R_{f} = 0.22 (Hexan: Ethylacetat; 2:1). FTIR (film): ṽ = 2912, 1752, 1611, 1511, 1383, 1241, 1031, 824, 754, 707 cm⁻¹. ¹H-NMR (400 MHz, DMSO-d₆): δ = 7.18 - 7.21 (m, 2H), 6.86 - 7.90 (m, 2H), 4.35 (t, *J* = 7.8 Hz, 2H), 4.05 (t, *J* = 5.5 Hz, 2H), 3.75 (s, 3H), 2.76 - 2.85 (m, 2H), 2.55 - 2.61 (m, 2H), 1.95 (s, 3H) ppm. ¹³C-NMR (125.8 MHz, DMSO-d₆): δ = 174.3 (C_{quart}), 157.9 (C_{quart}), 155.5 (C_{quart}), 133.3 (C_{quart}), 129.5 (2 CH), 114.0 (2 CH), 62.1 (CH₂), 55.3 (CH₃), 48.5 (CH₂), 40.5 (CH₂), 31.3 (CH₂), 19.2 (CH₃) ppm. MS (ESI, positive ion): 263([M+H]⁺, 100%).

### Beispiel 4: Methyl-{(3-[(2-oxo-1,3-oxazolidin-3-yl)imino]-2-pentylcyclopentyl}acetat (D)

In einen 250 mL-Einhalskolben wurden 0.60 g 3-Amino-1,3-oxazolidin-2-on in 50 mL Toluol vorgelegt. 2.95 g Hedion und 0.04 g *p*-Toluolsulfonsäure Monohydrat wurden hinzugefügt und das Gemisch bei 125 °C bis zum vollständigen Umsatz (3 h) gerührt. Anschließend wurde auf Raumtemperatur abgekühlt, das Lösungsmittel unter vermindertem Druck entfernt und säulenchromatographisch (Laufmittel Hexan:Ethylacetat) aufgereinigt. Das gewünschte Produkt **(D)** wurde als gelbliches Öl erhalten (m = 0.52 g, 34% über zwei Stufen). R_{f} = 0.14 (Hexan: Ethylacetat; 2:1). FTIR (film): ṽ = 2930, 1734, 1384, 1188, 1033, 753, 700 cm⁻¹. ¹H-NMR (400 MHz, DMSO-d₆): δ = 4.30 - 4.38 (m, 2H), 3.70 - 3.79 (m, 2H), 3.60 (s, 3H), 2.49 - 2.52 (m, 2H), 2.30 - 2.37 (m, 2H), 2.10 - 2.18 (m, 1H), 1.95 - 2.02 (m, 1H), 1.45 - 1.55 (m, 2H), 1.15 - 1.35 (m, 8H), 0.85 (t, *J* = 7.2 Hz, 3H) ppm. ¹³C-NMR (125.8 MHz, DMSO-d₆): δ = 182.5 (C_{quart}), 172.7 (C_{quart}), 155.2 (C_{quart}), 62.0 (CH₂), 51.7 (CH₃), 49.6 (CH), 48.0 (CH₂), 38.1 (CH), 37.0 (CH₂), 33.3 (CH₂), 31.9 (CH₂), 31.7 (CH₂), 28.5 (CH₂), 26.1 (CH₂), 22.3 (CH₂), 14.2 (CH₃) ppm. MS (ESI, positive ion): 311 ([M+H]⁺, 100%).

### Beispiel 5: 3-{[4-Phenylbutan-2-yliden]amino}-1,3-oxazolidin-2-on (E)

In einen 250 mL-Einhalskolben wurden 0.60 g 3-Amino-1,3-oxazolidin-2-on in 50 mL Toluol vorgelegt. 2.00 g Benzylaceton und 0.04 g p-Toluolsulfonsäure Monohydrat wurden hinzugefügt und das Gemisch bei 125 °C bis zum vollständigen Umsatz (3 h) gerührt. Anschließend wurde auf Raumtemperatur abgekühlt, das Lösungsmittel unter vermindertem Druck entfernt und säulenchromatographisch (Laufmittel Hexan:Ethylacetat) aufgereinigt. Das gewünschte Produkt **(E)** wurde als gelbliches Öl erhalten (m = 0.45 g, 38% über zwei Stufen). R_{f} = 0.33 (Hexan: Ethylacetat; 2:1). ¹H-NMR (400 MHz, DMSO-d₆): δ = 7.12 - 7.21 (m, 5H), 4.38 (t, *J* = 7.6 Hz, 2H), 3.75 (t, *J* = 7.8 Hz, 2H), 2.89 - 2.92 (m, 2H), 2.65 - 2.71 (m, 2H), 2.02 (s, 3H) ppm. ¹³C-NMR (125.8 MHz, DMSO-d₆): δ = 175.4 (C_{quart}), 155.9 (C_{quart}), 141.4 (C_{quart}), 128.7 (4 CH), 126.5 (CH), 62.2 (CH₂), 49.1 (CH₂), 40.9 (CH₂), 32.9 (CH₂), 17.9 (CH₃) ppm. MS (ESI, positive ion): 233 ([M+H]⁺, 100%).

### Beispiel 6: 3-{[7-Methyl-2H-1,5-benzodioxepin-3(4H)-yliden]amino}-1,3-oxazolidin-2-on (F)

In einen 250 mL-Einhalskolben wurden 0.70 g 3-Amino-1,3-oxazolidin-2-on in 50 mL Toluol vorgelegt. 2.70 g Calone und 0.04 g p-Toluolsulfonsäure Monohydrat wurden hinzugefügt und das Gemisch bei 125 °C bis zum vollständigen Umsatz (3 h) gerührt. Anschließend wurde auf Raumtemperatur abgekühlt, das Lösungsmittel unter vermindertem Druck entfernt und säulenchromatographisch (Laufmittel Hexan:Ethylacetat) aufgereinigt. Das gewünschte Produkt **(F)** wurde als gelbliches Öl erhalten (m = 0.33 g, 26% über zwei Stufen). R_{f} = 0.40 (Hexan: Ethylacetat; 2:1). FTIR (film): ṽ = 2920, 1749, 1503, 1386, 1184, 1019, 801, 747, 703, 588 cm⁻¹. ¹H-NMR (400 MHz, DMSO-d₆): δ = 6.85 - 6.90 (m, 1H), 6.72 - 6.78 (m, 1H), 6.68 - 6.71 (m, 1H), 4.95 (d, *J* = 9.5 Hz, 2H), 4.85 (d, *J* = 5.7 Hz, 2H), 4.45 (t, *J* = 7.5 Hz, 2H), 3.85 (t, *J* = 7.6 Hz, 2H), 2.20 (s, 3H) ppm. ¹³C-NMR (125.8 MHz, DMSO-d₆): δ = 167.9 (C_{quart}), 155.2 (C_{quart}), 147.9 (C_{quart}), 134.0 (C_{quart}), 132.5 (C_{quart}), 124.9 (CH), 122.0 (CH), 120.0 (CH), 71.5 (CH₂), 70.6 (CH₂), 62.6 (CH₂), 48.9 (CH₂), 20.5 (CH₃) ppm. MS (ESI, positive ion): 263 ([M+H]⁺, 100%).

### Beispiel 7: 3-[Decylideneamino]-1,3-oxazolidin-2-on (G)

In einen 250 mL-Einhalskolben wurden 0.60 g 3-Amino-1,3-oxazolidin-2-on aus Beispiel 1 in 50 mL Toluol vorgelegt. 2.00 g Decanal und 0.04 g p-Toluolsulfonsäure Monohydrat wurden hinzugefügt und das Gemisch bei 125 °C bis zum vollständigen Umsatz (2 h) gerührt. Anschließend wurde auf Raumtemperatur abgekühlt, das Lösungsmittel unter vermindertem Druck entfernt und säulenchromatographisch (Laufmittel Hexan:Ethylacetat) aufgereinigt. Das gewünschte Produkt **(G)** wurde als gelbliches Öl erhalten (m = 0.57 g, 48%, über zwei Stufen). R_{f} = 0.45 (Hexan: Ethylacetat; 1:1). FTIR (film): ṽ = 2923, 1753, 1407, 1224, 1098, 1039, 754 cm¹. ¹H-NMR (400 MHz, DMSO-d₆): δ = 7.05 (t, *J* = 5.4 Hz, 1H), 4.40 (t, *J* = 7.7 Hz, 2H), 3.73 (t, *J* = 7.7 Hz, 2H), 2.25 (q, *J* = 7.6 Hz, 2H), 1.15 (bs, 14H), 0.85 (t, *J* = 7.2 Hz, 3H) ppm. ¹³C-NMR (125.8 MHz, DMSO-d₆): δ = 154.3 (C_{quart}), 148.0 (CH), 61.7 (CH₂), 42.5 (CH₂), 32.4 (CH₂), 31.7 (CH₂), 29.6 (CH₂), 29.5 (CH₂), 29.3 (CH₂), 29.1 (CH₂), 26.6 (CH₂), 22.5 (CH₂), 14.2 (CH₃) ppm. MS (ESI, positive ion): 241 ([M+H]⁺, 100%).

### Beispiel 8: 3-({(2-methyl-3-[4-(propan-2-yl)phenyl]propyliden}amino)-1,3-oxazolidin-2-on (H)

In einen 250 mL-Einhalskolben wurden 0.70 g 3-Amino-1,3-oxazolidin-2-on in 50 mL Toluol vorgelegt. 2.90 g Cyclamenaldehyd und 0.04 g p-Toluolsulfonsäure Monohydrat wurden hinzugefügt und das Gemisch bei 125 °C bis zum vollständigen Umsatz (3 h) gerührt. Anschließend wurde auf Raumtemperatur abgekühlt, das Lösungsmittel unter vermindertem Druck entfernt und säulenchromatographisch (Laufmittel Hexan:Ethylacetat) aufgereinigt. Das gewünschte Produkt wurde als farbloser Feststoff **(H)** erhalten (m = 0.43 g, 32% über zwei Stufen). R_{f} = 0.12 (Hexan: Ethylacetat; 2:1). FTIR (film): ṽ = 2960, 1748, 1412, 1242, 1095, 1040, 754 cm⁻¹. ¹H-NMR (400 MHz, DMSO-d₆): δ = 7.10 - 7.16 (m, 4H), 7.08 (d, *J* = 5.7 Hz, 1H), 4.42 (t, *J* = 7.8 Hz, 2H), 3.68 - 3.72 (m, 2H), 2.80 - 2.88 (m, 2H), 2.62 - 2.72 (m, 1H), 2.55 - 2.60 (m, 1H), 1.20 (d, *J* = 6.8 Hz, 6H), 1.05 (d, *J* = 6.8 Hz, 3H) ppm. ¹³C-NMR (125.8 MHz, DMSO-d₆): δ = 154.3 (CH), 151.6 (C_{quart}), 146.3 (C_{quart}), 137.2 (C_{quart}), 129.2 (2 CH), 126.3 (2 CH), 61.7 (CH₂), 42.2 (CH₂), 39.7 (CH₂), 38.2 (CH), 33.4 (CH), 24.2 (2 CH₃), 17.7 (CH₃) ppm. MS (ESI, positive ion): 275 ([M+H]⁺, 100%).

### Beispiel 9: 3-{[2,6-dimethylhept-5-en-1-yliden]amino}-1,3-oxazolidin-2-on (I)

In einen 250 mL-Einhalskolben wurden 0.70 g 3-Amino-1,3-oxazolidin-2-on in 50 mL Toluol vorgelegt. 2.13 g Melonal und 0.04 g *p*-Toluolsulfonsäure Monohydrat wurden hinzugefügt und das Gemisch bei 125 °C bis zum vollständigen Umsatz (3 h) gerührt. Anschließend wurde auf Raumtemperatur abgekühlt, das Lösungsmittel unter vermindertem Druck entfernt und säulenchromatographisch (Laufmittel Hexan:Ethylacetat) aufgereinigt. Das gewünschte Produkt (**I**) wurde als gelbliches Öl erhalten (m = 0.36 g, 32% über zwei Stufen). R_{f} = 0.25 (Hexan: Ethylacetat; 2:1). ¹H-NMR (400 MHz, DMSO-d₆): δ = 6.95 (d, *J* = 6.0 Hz, 1H), 5.10 (t, *J* = 7.3 Hz, 1H), 4.42 (t, *J* = 7.5 Hz, 2H), 3.73 (t, *J* = 7.0 Hz, 2H), 2.35 - 2.45 (m, 1H), 1.95 - 1.98 (m, 2H), 1.65 (s, 3H), 1.58 (s, 3H), 1.49 - 1.51 (m, 1H), 1.35 - 1.43 (m, 1H), 1.05 (d, *J* = 6.9 Hz, 3H) ppm.¹³C-NMR (125.8 MHz, DMSO-d₆): δ = 154.4 (C_{quart}), 152.1 (CH), 131.4 (C_{quart}), 124.4 (CH), 61.7 (CH₂), 42.2 (CH₂), 36.3 (CH), 31.1 (CH₂), 25.8 (CH₃), 25.5 (CH₂), 18.1 (CH₃), 17.9 (CH₃) ppm. MS (ESI, positive ion): 225 ([M+H]⁺, 100%).

### Beispiel 10: 3-{[(4-Methoxyphenyl)methyliden]amino}-1,3-oxazolidin-2-on (J)

In einen 250 mL-Zweihalskolben wurden unter Stickstoffatmosphäre 1.50 g 3-Amino-1,3-oxazolidin-2-on aus Beispiel 1 in 50 mL Toluol vorgelegt. 3.00 g Anisaldehyd und 0.06 g p-Toluolsulfonsäure Monohydrat wurden hinzugefügt und das Gemisch bei 125 °C bis zum vollständigen Umsatz (3 h) gerührt. Anschließend wurde auf Raumtemperatur abgekühlt, das Lösungsmittel unter vermindertem Druck entfernt und säulenchromatographisch (Laufmittel Hexan:Ethylacetat) aufgereinigt. Das gewünschte Produkt **(J)** wurde farbloser Feststoff erhalten (m = 0.52 g, 48 % über zwei Stufen). R_{f} = 0.12 (Hexan: Ethylacetat; 2:1). FTIR (film): ṽ = 2917, 1752, 1605, 1471, 1414,1243, 1090, 1023, 829, 749, 531 cm⁻¹. ¹H-NMR (400 MHz, DMSO-d₆): δ = 7.68 - 7.71 (m, 2H), 7.28 - 7.31 (m, 2H), 6.95 - 7.00 (m, 1H), 4.39 (t, *J* = 7.3 Hz, 2H), 3.94 (t, *J* = 7.7 Hz, 2H), 3.45 (s, 3H) ppm. ¹³C-NMR (125.8 MHz, DMSO-d₆): δ = 160.9 (C_{quart}), 154.4 (C_{quart}), 143.8 (CH), 128.9 (CH), 127.3 (C_{quart}), 114.7 (CH), 61.9 (CH₂), 55.5 (CH₃), 42.5 (CH₂) ppm. MS (ESI, positive ion): 221 ([M+H]⁺, 100%).

### Beispiel 11: Riechtest

Nachfolgende Werte sind der Mittelwert aus den Bewertungen mindestens dreier Tester. Die Proben wurden wie folgt hergestellt: Es wurde eine ca. 200 mM Lösung der entsprechenden reinen Riechstoffe sowie deren Precursor in Diethylether oder Ethanol hergestellt. Anschließend wurde die Lösung auf Papierteststreifen aufgetragen, indem diese in die vorgefertigte Lösung eingetaucht wurde. Nachdem die Lösungsmittelfront etwa 2-3 cm nach oben gestiegen war, wurde der Teststreifen zum Trocknen bei Raumtemperatur auf einem Labortablett abgelegt und nach der angegebenen Zeit direkt abgerochen oder zuerst mit Wassernebel bzw. einer pH = 3 gepufferten Lösung besprüht und anschließend abgerochen. Skala: 0: Kein Geruch wahrnehmbar; 6: Sehr starker Duftreindruck.

| **Lösungs -mittel** | **Verbindung** | **15 min** | **6h** | **6h H20** | **6h pH 3** | **24h** | **24h H20** | **24h pH 3** | **48h** | **48h H2 O** | **48h pH 3** | **72h** | **144 h** |
|---|---|---|---|---|---|---|---|---|---|---|---|---|---|
| **EtOH** | β-Dihydrojonon | 6,00 | | | | 0,67 | | | 0,34 | | | | |
| | Dihydro-beta-jonon-Precursor **(B)** | 3,75 | | | | 1,17 | | | 0,34 | | | | |
| | Frambinonmethylether | 2,67 | | 2,17 | | | | | 1,67 | | | | 0,00 |
| | Frambinonmethylether Precursor **(C)** | 4,34 | | 2,34 | | | | | 1,83 | | | | 0,50 |
| | Hedion | 1,00 | 1,00 | 0,83 | 1,34 | 0,67 | 1,00 | 0,50 | 0,67 | 0,50 | 0,83 | 0,34 | 0,00 |
| | Hedion-Precursor **(D)** | 3,34 | 2,75 | 2,83 | 2,34 | 3,50 | 2,67 | 2,00 | 2,34 | 3,00 | 2,34 | 1,50 | 1,17 |
| | Benzylaceton | 5,00 | 1,00 | | | 0,00 | | | 0,75 | | | 0,00 | |
| | Benzylaceton - Precursor **(E)** | 3,00 | 4,00 | | | 2,33 | | | 2,75 | | | 1,25 | |
| | Calone | 5,00 | | | | | | 3,00 | | 1,75 | | | |
| | Calone - Precursor **(F)** | 1,00 | | | | | | 3,17 | | 1,83 | | | |
| | Decanal | 6,00 | | | | 2,00 | | | | | | 0,00 | |
| | Decanal Precursor **(G)** | 4,17 | | | | 2,67 | | | | | | 1,50 | |
| | Cyclamenaldehyd | 5,33 | | | 1,33 | | | | | | | | |
| | Cyclamenaldehyd - Precursor **(H)** | 2,17 | | | 2,50 | | | | | | | | |
| **Et₂O** | β-Dihydrojonon | 5,67 | | | | 1,17 | | | 0,67 | | | 0,17 | 0,00 |
| | Dihydro-beta-jonon-Precursor **(B)** | 3,34 | | | | 2,34 | | | 1,00 | | | 0,67 | 0,75 |
| | Frambinonmethylether | 3,34 | | | 1,50 | | | | | | | | |
| | Frambinonmethylether Precursor **(C)** | 2,67 | | | 2,17 | | | | | | | | |
| | Hedion | 1,67 | | | | 0,67 | 1,50 | 1,50 | 1,00 | 1,67 | 1,67 | 0,50 | 0,00 |
| | Hedion-Precursor **(D)** | 4,50 | | | | 3,00 | 2,67 | 2,83 | 1,84 | 1,84 | 2,67 | 1,50 | 1,50 |
| | Benzylaceton | 4,67 | | | | 0,30 | | | 2,25 | 1,75 | | 0,00 | |
| | Benzylaceton - Precursor **(E)** | 3,67 | | | | 3,67 | | | 4,00 | 2,75 | | 3,00 | |
| | Calone | 5,00 | | | | | | | | | 1,50 | | |
| | Calone - Precursor **(F)** | 2,67 | | | | | | | | | 2,50 | | |
| | Decanal | 6,00 | 1,67 | 1,33 | 1,00 | 2,67 | 0,50 | | 0,25 | 1,50 | 2,25 | 0,00 | |
| | Decanal Precursor **(G)** | 4,00 | 3,17 | 2,33 | 3,33 | 3,67 | 1,67 | | 1,25 | 2,75 | 2,75 | 3,00 | |
| | Melonal | 3,67 | | 0,33 | | | | | 0,67 | | | | |
| | Melonal - Precursor **(I)** | 2,33 | | 0,33 | | | | | 1,33 | | | | |

## Patentansprüche

1. Eine Duftvorläuferverbindung der Formel (I) 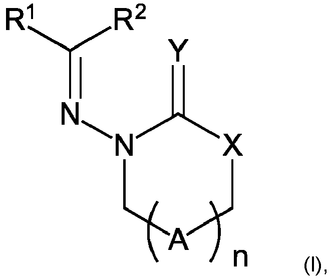 wobei
i) R¹ und R² unabhängig voneinander ausgewählt sind aus H, oder linearen, verzweigten oder cyclischen, gesättigten oder ungesättigten, substituierten oder unsubstituierten Kohlenwasserstoffresten, die bis zu 20 Kohlenstoffatome und gegebenenfalls bis zu 6 Heteroatome enthalten;
oder
ii) R¹ und R² gemeinsam mit dem Kohlenstoffatom an das sie gebunden sind einen cyclischen, gesättigten oder ungesättigten, Kohlenwasserstoffring mit bis zu 12 Kohlenstoffatomen bilden, der bis zu 6 Heteroatome enthalten kann;
X und Y unabhängig voneinander ausgewählt sind aus O, S, Se oder NR³, wobei R³ H oder ein linearer oder verzweigter, gesättigter oder ungesättigter, substituierter oder unsubstituierter Kohlenwasserstoffrest mit bis zu 20 Kohlenstoffatomen ist, der gegebenenfalls bis zu 6 Heteroatome enthalten kann;
A gleich -CH₂-, -CHR⁴-, -CR⁴R⁵- oder ein Heteroatom ist, wobei R⁴ und R⁵ unabhängig voneinander ausgewählt sind aus linearen oder verzweigten, gesättigten oder ungesättigten, substituierten oder unsubstituierten Kohlenwasserstoffresten, die bis zu 20 Kohlenstoffatome und gegebenenfalls bis zu 6 Heteroatome enthalten, wobei falls mehrere Einheiten A vorhanden sind, diese unabhängig voneinander ausgewählt werden;
n = 0 bis 4 ist,
mit der Maßgabe, dass sich der Rest R¹-C-R² von einem Duftstoffaldehyd oder einem Duftstoffketon der Formel R¹-C(=O)-R² ableitet.

2. Die Duftvorläuferverbindung nach Anspruch 1, wobei die mindestens eine Duftvorläuferverbindung der Formel (I) mindestens eine Duftvorläuferverbindung der Formel (II) ist 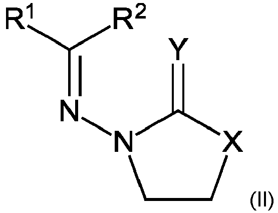 wobei
i) R¹ und R² unabhängig voneinander ausgewählt sind aus H, oder linearen, verzweigten oder cyclischen, gesättigten oder ungesättigten, substituierten oder unsubstituierten Kohlenwasserstoffresten, die bis zu 20 Kohlenstoffatome und gegebenenfalls bis zu 6 Heteroatome enthalten;
oder
ii) R¹ und R² gemeinsam mit dem Kohlenstoffatom an das sie gebunden sind einen cyclischen, gesättigten oder ungesättigten, Kohlenwasserstoffring mit bis zu 12 Kohlenstoffatomen bilden, der bis zu 6 Heteroatome enthalten kann;
X und Y unabhängig voneinander ausgewählt sind aus O, S, Se oder NR³, wobei R³ H oder ein linearer oder verzweigter, gesättigter oder ungesättigter, substituierter oder unsubstituierter Kohlenwasserstoffrest mit bis zu 20 Kohlenstoffatomen ist, der gegebenenfalls bis zu 6 Heteroatome enthalten kann, bevorzugt aus O oder S;
mit der Maßgabe, dass sich der Rest R¹-C-R² von einem Duftstoffaldehyd oder einem Duftstoffketon der Formel R¹-C(=O)-R² ableitet.

3. Die Duftvorläuferverbindung nach Anspruch 1 oder 2, wobei die mindestens eine Duftvorläuferverbindung der Formel (I) mindestens eine Duftvorläuferverbindung der Formel (III) ist 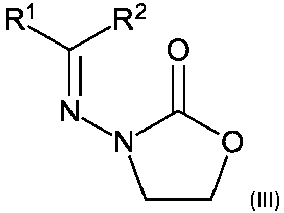 wobei
i) R¹ und R² unabhängig voneinander ausgewählt sind aus H, oder linearen, verzweigten oder cyclischen, gesättigten oder ungesättigten, substituierten oder unsubstituierten Kohlenwasserstoffresten, die bis zu 20 Kohlenstoffatome und gegebenenfalls bis zu 6 Heteroatome enthalten;
oder
ii) R¹ und R² gemeinsam mit dem Kohlenstoffatom an das sie gebunden sind einen cyclischen, gesättigten oder ungesättigten, Kohlenwasserstoffring mit bis zu 12 Kohlenstoffatomen bilden, der bis zu 6 Heteroatome enthalten kann;
mit der Maßgabe, dass sich der Rest R¹-C-R² von einem Duftstoffaldehyd oder einem Duftstoffketon der Formel R¹-C(=O)-R² ableitet.

4. Die Duftvorläuferverbindung nach einem der Ansprüche 1 bis 3, wobei die Verbindung durch die Umsetzung eines Hydrazids, bevorzugt 3-Amino-1,3-oxazolidin-2-on, mit mindestens einem Duftstoffaldehyd oder -keton ausgewählt aus Dihydro-beta-ionon, Frambinonmethylether, Hedion, Benzylaceton, Calone, Decanal, Cyclamenaldehyd, Melonal und Anisaldehyd oder Mischungen davon erhalten wird.

5. Wasch- oder Reinigungsmittel, enthaltend mindestens eine Duftvorläuferverbindung nach einem der Ansprüche 1 bis 4, wobei die Verbindung vorzugsweise in einer Gesamtmenge von 0,001 und 5 Gew.-%, vorteilhafterweise von 0,005 und 4 Gew.-%, weiter vorteilhaft von 0,01 und 2 Gew.-%, jeweils bezogen auf das Gesamtgewicht des Mittels, enthalten ist.

6. Wasch- oder Reinigungsmittel nach Anspruch 5, **dadurch gekennzeichnet, dass**
(1) es mindestens ein Tensid ausgewählt aus anionischen, kationischen, nichtionischen, zwitterionischen, amphoteren Tensiden oder Mischungen daraus enthält;
und/oder
(2) es in fester oder flüssiger Form vorliegt.

7. Luftpflegemittel, enthaltend mindestens eine Duftvorläuferverbindung nach einem der Ansprüche 1 bis 4, wobei die Verbindung vorzugsweise in einer Gesamtmenge von 0,001 und 5 Gew.-%, vorteilhafterweise von 0,005 und 4 Gew.-%, weiter vorteilhaft von 0,01 und 2 Gew.-%, bezogen auf das Gesamtgewicht des Mittels, enthalten ist.

8. Kosmetisches Mittel, enthaltend mindestens eine Duftvorläuferverbindung nach einem der Ansprüche 1 bis 4, wobei die Verbindung vorzugsweise in einer Gesamtmenge von 0,001 und 5 Gew.-%, vorteilhafterweise von 0,005 und 4 Gew.-%, weiter vorteilhaft von 0,01 und 2 Gew.-%, bezogen auf das Gesamtgewicht des Mittels, enthalten ist.

9. Verfahren zur Herstellung von Duftvorläuferverbindungen der Formeln (I) wie in Anspruch 1 definiert, umfassend das Umsetzen
einer Verbindung nach der allgemeinen Formel (IV) 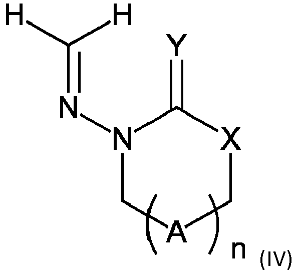 wobei Y, X, A und n wie in Formel (I) von Anspruch 1 definiert sind, mit Aldehyden der Formel R¹-C(=O)H, Ketonen der Formel R¹-C(=O)-R² oder Mischungen davon, wobei R¹ und R² wie in Formel (I) definiert sind.

10. Verfahren zur langanhaltenden Beduftung von Oberflächen, **dadurch gekennzeichnet, dass** eine Duftvorläuferverbindung nach einem der Ansprüche 1 bis 4 oder ein Mittel nach Anspruch 5, 6 oder 8 auf die zu beduftende Oberfläche aufgebracht wird, wobei die Beduftung länger anhält als würde die jeweilige Duftstoffverbindung oder ein identisches Mittel in dem die Duftvorläuferverbindung durch die jeweilige Duftstoffverbindung ersetzt ist eingesetzt werden.
